# EUROPEAN PATENT SPECIFICATION

(11) **EP 1 286 591 B1**
(45) Date of publication and mention of the grant of the patent: **06.10.2010**
(21) Application number: 00941235.4
(22) Date of filing: 07.06.2000
(51) Int. Cl.: A61K 31/216, A61K 31/505, A61P 1/06, A61P 13/00

(54) **TREATING SMOOTH MUSCLE HYPERACTIVITY WITH (R)-OXYBUTYNIN AND (R)- DESETHYLOXYBUTYNIN**
BEHANDLUNG VON ÜBERAKTIVITÄT GLATTER MUSKULATUR MIT (R)-OXYBUTYNIN UND (R)-DESETHYLOXYBUTYNIN
TRAITEMENT DE L'HYPERACTIVITE DE MUSCLES LISSES AVEC (R)-OXYBUTYNINE ET (R)-DESETHYLOXYBUTYNINE

(43) Date of publication of application: 05.03.2003
(73) Proprietor: Watson Pharmaceuticals, Inc., Corona, CA 32880-2882 (US)
(72) Inventor: ABERG, A., K. Gunnar, Sarasota, FL 34242 (US)
(74) Representative: Thomson, Paul Anthony
(86) International application number: PCT/US2000/015515
(87) International publication number: WO 2001/093683

(56) References cited:
- WO-A-96/23492
- US-A- 4 008 269
- US-A- 4 973 592
- US-A- 5 973 182
- WALLIS R M ET AL: "MUSCARINIC ANTAGONISTS IN DEVELOPMENT FOR DISORDERS OF SMOOTH MUSCLE FUNCTION" LIFE SCIENCES, PERGAMON PRESS, OXFORD, GB, vol. 64, no. 6/7, 8 January 1999 (1999-01-08), pages 395-401, XP000972297 ISSN: 0024-3205
- SMITH E R ET AL: "COMPARISON OF THE ANTIMUSCARINIC AND ANTISPASMODIC ACTIONS OF RACEMIC OXYBUTYNIN AND DESETHYLOCYBUTYNIN AND THEIR ENANTIOMERS WITH THOSE OF RACEMIC TERODILINE" ARZNEIMITTEL FORSCHUNG. DRUG RESEARCH, ECV EDITIO CANTOR VERLAG, AULENDORF, DE, vol. 48, no. 10, October 1998 (1998-10), pages 1012-1018, XP008061986 ISSN: 0004-4172
- HUGHES K M ET AL: "MEASUREMENT OF OXYNUTYNIN AND ITS N-DESETHYL METABOLITE IN PLASMA, AND ITS APPLICATION TO PHARMACOKINETIC STUDIES IN YOUNG, ELDERLY AND FRAIL ELDERLY VOLUNTEERS" XENOBIOTICA, TAYLOR AND FRANCIS, LONDON,, GB, vol. 22, no. 7, 1 July 1992 (1992-07-01), pages 859-869, XP000572170 ISSN: 0049-8254
- NORONHA-BLOB L ET AL: "ENANTIOMERS OF OXYBUTYNIN: IN VITRO PHARMACOLOGICAL CHARACTERIZATION AT M1, M2 AND M3 MUSCARINIC RECEPTORS AND IN VIVO EFFECTS ON URINARY BLADDER CONTRACTION, MYDRIASIS AND SALIVARY SECRETION IN GUINEA PIGS" JOURNAL OF PHARMACOLOGY AND EXPERIMENTAL THERAPEUTICS, AMERICAN SOCIETY FOR PHARMACOLOGY AND, US, vol. 256, no. 2, 1 February 1991 (1991-02-01), pages 562-567, XP000572006 ISSN: 0022-3565

## Description

### FIELD OF THE INVENTION.

The invention relates to the optically active R(-)-isomer of 4-diethylamino-2-butynyl cyclohexylphenylglycolate and to the optically active metabolite thereof, called R(-)-4-ethylamino-2-butynyl cyclohexylphenylglycolate. The compound 4-diethylamino-2-butynyl cyclohexylphenylglycolate has the generic name oxybutynin (OXY) and is an approved drug for the management of urinary incontinence and is also useful for the treatment of gastrointestinal smooth muscle hyperactivity.

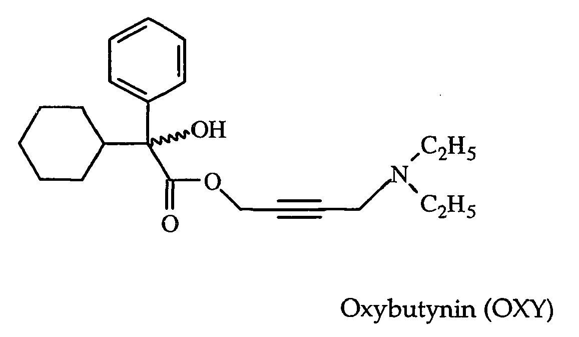

The compound 4-ethylamino-2-butynyl cyclohexylphenylglycolate is called desethyloxybutynin (DEO) and has the following chemical structure:

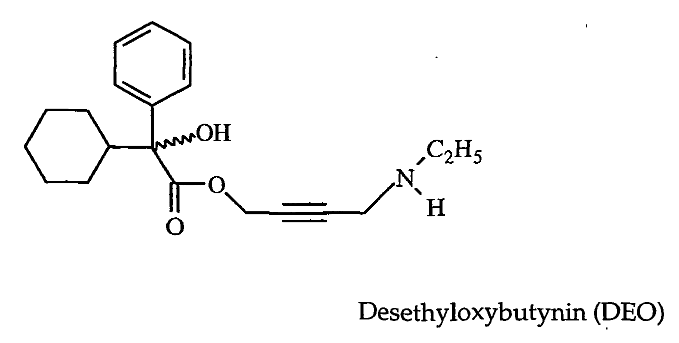

### BACKGROUND OF THE INVENTION.

Racemic oxybutynin (OXY) is used therapeutically in the treatment smooth muscle hyperactivity, such as for example urinary incontinence. OXY exerts a spasmolytic effect by inhibiting the receptors for acetylcholine on smooth muscle. OXY is selective for muscarinic acetylcholine receptors over nicotinic receptors and as a result, no blocking effects are observed at skeletal neuromuscular junctions.

In patients with conditions characterized by involuntary bladder contractions, clinical studies have demonstrated that OXY increases bladder capacity, diminishes the frequency of involuntary contractions of the detrusor muscle, and delays the initial desire to void. OXY is therefore useful in the treatment and prevention of both incontinency and frequent voluntary urination. Antimuscarinic side effects of OXY, such as mydriasis, xerostomia and tachycardia, cannot be avoided with this drug since therapeutic antimuscarinic effects are sought (Lish et al., 1965).

Racemic oxybutynin consists of a mixture of 50% R(-)-oxybutynin and 50% S(+)-oxybutynin. It has been shown that practically all of the anticholinergic activity of OXY resides in the R(-)-isomer, and the S(+)-isomer carries only a small fraction of the drug's anticholinergic activity (Noronha-Blob et al.,1991 and Smith et al. 1998.)

One clinically important metabolite of OXY has been identified in humans after administration of OXY and is called desethyloxybutynin (DEO). A second metabolite, called N-oxide-oxybutynin. N-oxide-oxybutynin is a therapeutically active compound but may not be chemically stable (Lindeke et al. 1981).

### SUMMARY OF THE INVENTION

It has now unexpectedly been found that the S(+)-isomers of oxybutynin and of desethyloxybutynin carry serious cardiovascular depressing activity of oxybutynin. This unwanted side effect is of concern in all patients given racemic oxybutynin and particularly in patients that are of age or patients that have pre-existing cardiovascular conditions. It was found that the R(-)-isomer of oxybutynin quite unexpectedly was much better tolerated than the S(+)-isomer, when given to laboratory animals. It has furthermore now been found that the R(-)-isomers of oxybutynin and of desethyloxybutynin provide superior therapeutic effects when compared to their corresponding racemates and their optical antipodes. Thus, the active compounds of the present invention are the R(-)-isomer of oxybutynin and the R(-)-isomer of desethyloxybutynin.

### CHEMISTRY

Racemic oxybutynin is 4-diethylamino-2-butynyl-cyclohexyl-hydroxy-benzeneacetate, also known as 4-diethylamino-2-butynyl cyclohexylphenylglycolate and herein also referred to as OXY. The generic name given to the hydrochloride salt of racemic oxybutynin by the USAN Council is oxybutynin chloride; it is sold under the name of Ditropan^{®}.

R(-)-oxybutynin is R(-)-4-diethylamino-2-butynyl_-cyclohexyl-_-hydroxy-benzeneacetate and herein also referred to as R-OXY. No generic name is known for this compound or any of its salts.

Racemic desethyloxybutynin is 4-ethylamino-2-butynyl cyclohexylphenyl-glycolate and is a known metabolite of oxybutynin (Hughes K.M. et al. 1992). This compound is herein also referred to as DEO. No generic name is known for this compound or any of its salts.

R(-)-desethyloxybutynin is R(-)-4-ethylamino-2-butynyl cyclohexylphenyl-glycolate and herein also referred to as R-DEO. No generic name is known for this compound or any of its salts.

The overall process for preparing R-OXY involves:
(a) the preparation of 4-diethylamino-2-butynyl chloride from dichlorobutyne
(b) by standard esterification technique, reacting the single R-enantiomer of cyclohexylphenylglycolic acid with the prepared 4-diethylamino-2-butynyl chloride to the R-enantiomer of 4-diethylamino-2-butynyl cyclohexylphenylglycolate.

The process for preparing R-DEO involves:
(a) the preparation of the side chain 4-ethylamino-2-butynyl chloride from dichlorobutyne
(b) by standard esterification technique, reacting the R-enantiomer of cyclohexylphenylglycolic acid with the selected side chain to produce the R(-)-enantiomer of 4-ethylamino-2-butynyl cyclohexylphenyl-glycolate.

Alternative processes for preparing the compounds of the invention involve the preparation of hydroxylated side chains in stead of the above mentioned halogenated side chains.

Racemic cyclohexylphenylglycolic acid is commercially available from SIPSY Chem Corp., 2137 Route 33, Suite 2, Hamilton Square, NJ 08690.

The R-enantiomer of cyclohexylphenylglycolic acid can be obtained by resolution of racemic cyclohexylphenylglycolic acid or by chiral synthesis.

R-OXY and R-DEO can also be prepared by the resolution of racemic material, using conventional means such as fractional crystallization of diastereomeric salts with chiral acids. Other standard methods of resolution known to those skilled in the art, include, but are not limited to, simple crystallization and chromatography on a chiral substrate, and can also be used.

### DOSING, DOSAGE FORMS, PHARMACEUTICAL COMPOSITIONS

The magnitude of a prophylactic or therapeutic dose of the compounds of this invention in the acute or chronic management of disease will vary with the severity and nature of the condition to be treated and the route of administration. The dose and the frequency of the dosing will also vary according to the age, body weight and response of the individual patient. In general, the total daily dose range for the compounds of this invention for the conditions described herein is from about 1 mg to about 100 mg in single or divided doses, preferably in divided doses. In managing the patient, the therapy should be initiated at a lower dose, perhaps at about 0.5 mg to about 25 mg, and may be increased up to about 200 mg depending on the patient's global response. It is further recommended that patients over 65 years and those with impaired renal or hepatic function initially receive low doses and that they be titrated based on individual response(s) and plasma drug level(s). It may be necessary to use dosages outside these ranges, as will be apparent to those skilled in the art. Further, it is noted that the clinician or treating physician will know how and when to interrupt, adjust, or terminate therapy in conjunction with individual patient response. The terms "a therapeutically effective amount" and "an amount sufficient to treat urinary incontinence but insufficient to cause adverse effects" are encompassed by the above-described dosage amounts and dose frequency schedule.

Any suitable route of administration may be employed for providing the patient with an effective dosage of the compounds of this invention. For example, oral, sublingual, rectal, parental (subcutaneous, intramuscular, intravenous), intraocular, transdermal, aerosol and like forms of administration may be employed. Dosage forms include tablets, troches, dispersions, suspensions, solutions, capsules, microencapsulated systems, sprays, transdermal delivery systems, and the like.

The pharmaceutical compositions of the present invention comprise a compound of the present invention as the active ingredient, or a pharmaceutically acceptable salt or solvate thereof, and may also contain a pharmaceutically acceptable carrier, and optionally, other therapeutic ingredients.

The terms "pharmaceutically acceptable salt(s) or solvate(s)" or "a pharmaceutically acceptable salt(s) or solvate(s) thereof" refer to salts or solvates prepared from pharmaceutically acceptable non-toxic acids. Suitable pharmaceutically acceptable acid addition salts for the compound of the present invention include acetic, benzenesulfonic (besylate), benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pathothenic, phosphoric, succinic, sulfuric, tartaric, p-toluenesulfonic, and the like. The hydrochloride is particularly preferred.

The compositions of the present invention include suspensions, solutions, elixirs or solid dosage forms. Carriers such as starches, sugars, and microcrystalline cellulose, diluents, granulating agents, lubricants, binders, disintegrating agents, and the like are suitable in the case of oral solid preparations (such as powders, capsules, and tablets), and oral solid preparations are preferred over the oral liquid preparations.

Because of their ease of administration, tablets and capsules represent advantageous oral dosage unit forms, in which case solid pharmaceutical carriers are employed. If desired, tablets may be coated by standard aqueous or nonaqueous techniques.

In addition to the common dosage forms set out above, the compounds of the present invention may also be administered by controlled release means and delivery devices such as those described in U.S. Patent Nos.: 3,845,770; 3,916,899; 3,536,809; 3,598,123; and 4,008,719, and PCT application WO92/20377.

Pharmaceutical compositions of the present invention suitable for oral administration may be presented as discrete unit dosage forms such as capsules, cachets, or tablets, each containing a predetermined amount of the active ingredient, as a powder or granules, or as a solution or a suspension in an aqueous liquid, a non-aqueous liquid, an oil-in-water emulsion, or a water-in-oil liquid emulsion. Such compositions may be prepared by any of the methods of pharmacy, but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation, just as is known for the racemic mixture.

For example, a tablet may be prepared by compression or molding, optionally, with one or more accessory ingredients. Compressed tablets may be prepared by compressing in a suitable machine the active ingredient in a free-flowing form such as powder or granules, optionally mixed with a binder, lubricant, inert diluent, surface active agent or dispersing agent. Molded tablets may be made by molding, in a suitable machine, a mixture of the powdered compound moistened with an inert liquid diluent. All of the foregoing techniques are well know to persons of skill in the pharmaceutical art. Each tablet may contain from about 0.5 mg to about 25 mg of the active ingredient.

### EXAMPLES

### Example 1

### ORAL UNIT DOSAGE FORMULATION

### Tablets:

| Ingredients | per tablet | per batch of 10,000 tablets |
|---|---|---|
| R-OXY or R-DEO | 5 mg | 50 g |
| Microcrystalline cellulose | 30 mg | 300 g |
| Lactose | 70 mg | 700 g |
| Calcium stearate | 2 mg | 20 g |
| FD&C Blue #1 Lake | 0.03 mg | 300 mg |

The selected compound of the present invention is blended with the lactose and cellulose until a uniform blend is formed. The lake is added and further blended. Finally, the calcium stearate is blended in, and the resulting mixture is compressed into tablets using a 9/32 inch (7 mm) shallow concave punch. Tablets of other strengths may be prepared by altering the ration of active ingredient to the excipients or to the final weight of the tablet.

The surprising utility of the compounds of the present invention has been established by the following studies.

### Example 2.

### Pharmacological Studies

### MATERIALS AND METHODS

### 1. Acute Toxicity in mice.

The experiments are carried out on conscious albino mice that are administered escalating doses of the test compounds intravenously or orally.

### 2. Ligand Binding Studies: Muscarinic Receptors.

The experiments are carried out on membranes prepared from SF9 cells infected with baculovirus to express human recombinant muscarinic receptor subtypes. After incubation with the test article and the proper radioligand and washing, bound radioactivity is determined with a liquid scintillation counter, using a commercial scintillation cocktail. The specific radioligand binding to each receptor is defined as the difference between total binding and nonspecific binding determined in the presence of an excess of unlabelled ligand. IC₅₀ values (concentrations required for inhibition of 50% of specific binding) are determined by non linear regression analysis of the competition curves. These parameters are obtained by curve fitting using Sigmaplot^{™} software.

### 3. Cardiac side effect studies.

Male guinea pigs (450-600 g) are anesthetized with freshly prepared dialurethane sodium. The jugular vein is catheterized for iv administration of test drugs and the trachea is exposed and cannulated. Subdermal electrodes are positioned for Lead II electrocardiogram recording, monitored on a Grass Polygraph recorder, set at a paper speed of 50 mm/sec. The animals are allowed to stabilize for 30 minute after completion of surgery, and three baseline EKG recordings are then made at 10-minute intervals. The animals are then given a dose of the test compound or vehicle as an intravenous infusion over 30 min. EKG recordings are used to determine QT intervals and heart rates. To compensate for variations in heart rates, QTc intervals are calculated from QT- and RR-intervals as known to those skilled in the art. Prolongation of QTc is indicative of a prolonged action potential, caused by an inhibition of the delayed rectifier potassium channel. Prolongation of QTc is the known cause of Torsades de Pointes ventricular fibrillation by drugs such as terodiline, terfenadine and astemizole (now withdrawn from the market).

### 4. Functional Characterization of Antimuscarinic/Antispasmodic Activity.

Strips of urinary bladder smooth muscle or of intestinal smooth muscle tissue are removed from the body of male Hartley guinea pigs weighing 400-600 g. Experiments are performed using methods similar to those described by Kachur et al, 1988 and Noronha-Blob and Kachur, 1991. Strips of bladder tissue (approximately 10 mm long and 1.5 mm wide) are removed from the body of the urinary bladder of male Hartley guinea pigs weighing 400-600 g. Preparations of the longitudinal smooth muscle of the colon of guinea pigs are prepared as known in the art (Acta Physiol Scand 64: 15-27,1965). The tissues are suspended in an oxygenated buffer of the following composition, in mM: NaCl, 133; KCI, 4.7; CaCl₂, 2.5; MgSO₄, 0.6; NaH₂PO₄, 1.3; NaHCO₃, 16.3; and glucose, 7.7, or of a similar composition. They are maintained at 37.5 C. Contractions are recorded with isometric transducers (Model FT-10) on an ink-writing polygraph.

In each experiment up to seven bladder strips or up to four intestinal smooth muscle strips are removed from a single animal, suspended in tissue chambers and allowed to equilibrate with the bathing solution for one hour before proceeding with the experiment.

In order to assess the viability of each tissue and to serve as a frame of reference, contractions of each strip of tissue are recorded initially in response to exposure to a tissue medium in which the NaCl was replaced by KCI to yield a concentration of 137.7 mM KCl in the medium. This is followed by return to the standard medium, and then by exposures to progressively creasing concentrations of carbachol, with separate exposures to each concentration only until the peak response has been recorded. Then, leaving one strip untreated and/or one strip exposed to the test solution to serve as control tissue(s), the remaining strips each are exposed for one hour to one concentration of an antagonist. Finally, the responses to increasing concentrations of carbachol followed by exposure to 137.7 mM KCl are recorded a second time. To determine whether antagonists decrease the peak response to agonists, the peak tension developed by each strip during the second set of determinations is expressed as a percent of the peak tension developed during the first concentration-effect determination. Then, for each antagonist the resultant data are analyzed using standard statistical methodology.

### EQUIVALENTS

Those skilled in the art will recognize, or be able to ascertain, using no more than routine experimentation, many equivalents to the specific embodiments of the invention described herein. Such equivalents include numerous pharmaceutically acceptable salt or solvate forms e.g. sulfate, fumarate, hydrobromide, hydrochloride, dihydrochloride, methanesulphonate, hydroxynaphthoate, chlorotheophylline or where appropriate one or other of the hydrate forms thereof, see Merck Index 11th edition (1989) items 9089, 209, 3927, 4628, 8223, 5053, 5836, 8142, 2347, 7765, 1840, 9720, 7461, 1317,4159, and 963 and references cited therein and Am. Rev. Resp. Dis. 1988,137: (4;2/2) 32. Such equivalents also include the co-administration of at least one compound of the present invention with any other drug that is used to combat diseases in mammals, mentioned in this document. Such equivalents also include the co-administration of at least one compound of the present invention with any other compound or drug that may be used in combination with medication for urinary incontinence or intestinal hyperactivity. Those skilled in the art of medicine will also realize that higher or lower doses than those indicated here may be preferred and the doses may be given more or less frequently than suggested here.

Those skilled in the art will realize that smooth muscle hyperactivity may be expressed in various types of smooth muscle i.e. those of the urinary bladder, the gastrointestinal tract, the kidneys and the gall bladder.

Those skilled in the art, will realize that intestinal hyperactivity disorders include irritable bowel syndromes (IBS), that urinary bladder hyperactivity disorders include urinary urge incontinence, and that hyperactivity of the kidney and the gall bladder and gall ducts include those causing kidney and gall stone pain.

Those skilled in the art of pharmacology, will realize that the compounds of the invention, having certain pharmacological properties (such as antimuscarinic activity on various receptor types, calcium antagonistic activity, spasmolytic activity on various types of smooth muscle etc.) may be useful for other indications than those listed here.

## Claims

1. Use of R(-)-4-diethylamino-2-butynyl cyclohexylphenylglycolate, including pharmaceutically acceptable salts or solvates thereof, in the manufacture of a pharmaceutical composition for treating urinary incontinence, and other motility disorders involving the urethrogenital tract, while reducing concomitant liability of adverse cardiovascular depressing effects associated with racemic oxybutynin administration, which comprises a therapeutically effective amount of R(-)-4-diethylamino-2-butynyl cyclohexylphenylglycolate or a pharmaceutically acceptable salt or solvate thereof, substantially free of its corresponding S-enantiomer.

2. Use of R(-)-4-diethylamino-2-butynyl cyclohexylphenylglycolate, including pharmaceutically acceptable salts or solvates thereof, in the manufacture of a pharmaceutical composition for treating irritable bowl syndromes (IBS), and Other motility disorders involving the gastrointestinal tract, while reduces concomitant liability of adverse cardiovascular depressing effects associated with racemic oxybutynin administration, which comprises a therapeutically effective amount of R(-)-4-diethylamino-2-burynyl cyclohexylphenylglycolate or a pharmaceutically acceptable salt or solvate thereof, substantially free of its corresponding S-enantiomer.

3. Use of R(-)-4-ethylamino-2-butynyl cyclohexylphenylglycolate, including pharmaceutically acceptable salts or solvates thereof, in the manufacture of a pharmaceutical composition for treating urinary incontinence, and other motility disorders involving the urethrogenital tract, while reducing concomitant liability of adverse cardiovascular depressing effects associated with racemic oxybutynin administration, which comprises a therapeutically effective amount of R(-)-4-ethylamino-2-butynyl cyclohexylphenylglycolate or a pharmaceutically acceptable salt or solvate thereof, substantially free of its corresponding S-enantiomer.

4. Use of R(-)-4-ethylamino-2-butynyl cyclohexylphenylglycolate, including pharmaceutically acceptable salts or solvates thereof, in the manufacture of a pharmaceutical composition for treating irritable bowel syndrome (IBS), and other motility disorders involving the gastrointestinal tract, while reducing concomitant liability of adverse cardiovascular depressing effects associated with racemic oxybutynin administration, which comprises a therapeutically effective amount of R(-)-4-ethylamino-2-butynyl cyclohexylphenylglycolate or a pharmaceutically acceptable salt or solvate thereof, substantially free of its corresponding S-enantiomer.

5. The use of claims 1 or 2 wherein the R(-)-4-diethylamino-2-butyynyl cyclohexylphenylglycolate, pharmaceutically acceptable salt, or solvate thereof, is configured for administration by a route selected from the group consisting of inhalation, parenteral, transdermal, rectal, sublingual, or oral routes of administration.

6. The use of claims 3 or 4 wherein the R(-)-4-ethylamino-2-butyynyl cyclohexylphenylglycolate, pharmaceutically acceptable salt, or solvate thereof, is configured for administration by a route selected from the group consisting of inhalation, parenteral, transdermal, rectal, sublingual, or oral routes of administration.

7. The use of claims 1 or 2 wherein the R(-)-4-diethylamino-2-butyynyl cyclohexylphenylglycolate, pharmaceutically acceptabte salt, or solvate thereof, is configured for oral administration.

8. The use of claims 3 or 4 wherein the R(-)4-ethylamino-2-butyynyl cyclohexylphenylglycolate, pharmaceutically acceptable salt, or solvate thereof, is configured for oral administration.

9. The use of claims 1 or 2 wherein R(-)-4-diethylamino-2-butyynyl cyclohexylphenylglycolate, pharmaceutically acceptable salt, or solvate thereof, is configured for extended release oral administration.

10. The use of claims 3 or 4 wherein the R(-)-4-ethylamino-2-butyynyl cyclohexylphenylglycolate, pharmaceutically acceptable salt, or solvate thereof, is configured for extended release oral administration.

11. The use of claims 1 or 2 wherein the R(-)-4-diethylamino-2-butyynyl cyclohexylphenylglycolate, pharmaceutically acceptable salt, or solvate thereof, is configured for transdermal administration.

12. The use of claims 3 or 4 wherein the R(-)-4-ethylamino-2-butyynyl cyclohexylphenylglycolate, pharmaceutically acceptable salt, or solvate thereof, is configured for transdermal administration.

13. The use of claims 1 or 2 wherein the amount of R(-)-4-diethylamino-2-butyynyl cyclohexylphenylglycolate, pharmaceutically acceptable salt, or solvate thereof, is sufficient to provide a dosage of from about 1 mg to about 200 mg per day.

14. The use of claims 3 or 4 wherein the amount of R(-)-4-ethylamino-2-butyynyl cyclohexylphenylglycolate, pharmaceutically acceptable salt, or solvate thereof, is sufficient to provide a dosage of from about 1 mg to about 200 mg per day.

## Patentansprüche

1. Verwendung eines R(-)-4-Diethylamin-2-Butynylcyclohexylphenylglycolats, einschließlich pharmazeutisch akzeptabler Salze oder Solvate davon, bei der Herstellung einer pharmazeutischen Zusammensetzung zur Behandlung von Urininkontinenz und anderen Motilitätsstörungen, die den Harnröhren- und Genitaltrakt betreffen, während die begleitende Belastung durch nachteilige kardiovaskuläre Unterdrückungseffekte im Zusammenhang mit der Verabreichung von racemischem Oxybutynin verringert wird, wobei die Zusammensetzung eine therapeutisch wirksame Menge an R(-)-4-Diethylamin-2-Butynylcyclohexylphenylglycolat oder eines pharmazeutisch akzeptablen Salz oder Solvats davon umfasst, das im Wesentlichen frei von seinem entsprechenden S-Enantiomer ist.

2. Verwendung eines R(-)-4-Diethylamin-2-Butynylcyclohexylphenylglycolats, einschließlich pharmazeutisch akzeptabler Salze oder Solvate davon, bei der Herstellung einer pharmazeutischen Zusammensetzung zur Behandlung des Reizdarmsyndroms und anderer Motilitätsstörungen, die den Magen-Darm-Trakt betreffen, während die begleitende Belastung durch nachteilige kardiovaskuläre Unterdrückungseffekte im Zusammenhang mit der Verabreichung von racemischem Oxybutynin verringert wird, wobei die Zusammensetzung eine therapeutisch wirksame Menge an R(-)-4-Diethylamin-2-Butynylcyclohexylphenylglycolat oder eines pharmazeutisch akzeptablen Salzes oder Solvats davon umfasst, das im Wesentlichen frei von seinem entsprechenden S-Enantiomer ist.

3. Verwendung eines R(-)-4-Diethylamin-2-Butynylcyclohexylphenylglycolats, einschließlich pharmazeutisch akzeptabler Salze oder Solvate davon, bei der Herstellung einer pharmazeutischen Zusammensetzung zur Behandlung von Urininkontinenz und anderen Motilitätsstörungen, die den Harnröhren- und Genitaltrakt betreffen, während die begleitende Belastung durch nachteilige kardiovaskuläre Unterdrückungseffekte im Zusammenhang mit der Verabreichung von racemischem Oxybutynin verringert wird, wobei die Zusammensetzung eine therapeutisch wirksame Menge an R(-)-4-Ethylamin-2-Butynylcyclohexylphenylglycolat oder eines pharmazeutisch akzeptablen Salzes oder Solvats davon umfasst, das im Wesentlichen frei von seinem entsprechenden S-Enantiomer ist.

4. Verwendung eines R(-)-4-Ethylamin-2-Butynylcyclohexylphenylglycolats, einschließlich pharmazeutisch akzeptabler Salze oder Solvate davon, bei der Herstellung einer pharmazeutischen Zusammensetzung zur Behandlung des Reizdarmsyndroms und anderer Motilitätsstörungen, die den Magen-Darm-Trakt betreffen, während die begleitende Belastung durch nachteilige kardiovaskuläre Unterdrückungseffekte im Zusammenhang mit der Verabreichung von racemischem Oxybutynin verringert wird, wobei die Zusammensetzung eine therapeutisch wirksame Menge an R(-)-4-Ethylamin-2-Butynylcyclohexylphenylglycolat oder eines pharmazeutisch akzeptablen Salzes oder Solvats davon umfasst, das im Wesentlichen frei von seinem entsprechenden S-Enantiomer ist.

5. Verwendung nach Anspruch 1 oder 2, wobei das R(-)-4-Diethylamin-2-Butynylcyclohexylphenylglycolat, das pharmazeutisch akzeptable Salz oder Solvat davon für die Verabreichung über einen Weg konfiguriert ist, der aus der Gruppe ausgewählt wird, die aus Folgendem besteht: Inhalation, parenteraler, transdermaler, rektaler, sublingualer oder oraler Verabreichungsweg.

6. Verwendung nach Anspruch 3 oder 4, wobei das R(-)-4-Ethylamin-2-Butynylcyclohexylphenylglycolat, das pharmazeutisch akzeptable Salz oder Solvat davon für die Verabreichung über einen Weg konfiguriert ist, der aus der Gruppe ausgewählt wird, die aus Folgendem besteht: Inhalation, parenteraler, transdermaler, rektaler, sublingualer oder oraler Verabreichungsweg.

7. Verwendung nach Anspruch 1 oder 2, wobei das R(-)-4-Diethylamin-2-Butynylcyclohexylphenylglycolat, das pharmazeutisch akzeptable Salz oder Solvat davon für die orale Verabreichung konfiguriert ist.

8. Verwendung nach Anspruch 3 oder 4, wobei das R(-)-4-Ethylamin-2-Butynylcyclohexylphenylglycolat, das pharmazeutisch akzeptable Salz oder Solvat davon für die orale Verabreichung konfiguriert ist.

9. Verwendung nach Anspruch 1 oder 2, wobei das R(-)-4-Diethylamin-2-Butynylcyclohexylphenylglycolat, das pharmazeutisch akzeptable Salz oder Solvat davon für die orale Verabreichung mit verlängerter Freigabe konfiguriert ist.

10. Verwendung nach Anspruch 3 oder 4, wobei das R(-)-4-Ethylamin-2-Butynylcyclohexylphenylglycolat, das pharmazeutisch akzeptable Salz oder Solvat davon für die orale Verabreichung mit verlängerter Freigabe konfiguriert ist.

11. Verwendung nach Anspruch 1 oder 2, wobei das R(-)-4-Diethylamin-2-Butynylcyclohexylphenylglycolat, das pharmazeutisch akzeptable Salz oder Solvat davon für die transdermale Verabreichung konfiguriert ist.

12. Verwendung nach Anspruch 3 oder 4, wobei das R(-)-4-Ethylamin-2-Butynylcyclohexylphenylglycolat, das pharmazeutisch akzeptable Salz oder Solvat davon für die transdermale Verabreichung konfiguriert ist.

13. Verwendung nach Anspruch 1 oder 2, wobei die Menge des R(-)-4-Diethylamin-2-Butynylcyclohexylphenylglycolats, des pharmazeutisch akzeptablen Salzes oder Solvats davon für die Bereitstellung einer Dosierung von etwa 1 mg bis etwa 200 mg pro Tag ausreicht.

14. Verwendung nach Anspruch 3 oder 4, wobei die Menge des R(-)-4-Ethylamin-2-Butynylcyclohexylphenylglycolats, des pharmazeutisch akzeptablen Salzes oder Solvats davon für die Bereitstellung einer Dosierung von etwa 1 mg bis etwa 200 mg pro Tag ausreicht.

## Revendications

1. Utilisation de R(-)-4-diéthylamino-2-butynyle cyclohexylphénylglycolate, y compris des sels de qualité pharmaceutique ou des solvates de ces derniers, dans la fabrication d'une composition pharmaceutique pour traiter l'incontinence urinaire, et autres troubles de motilité impliquant le système urétrogénital, tout en réduisant le risque concomitant d'effets inhibiteurs cardiovasculaires indésirables associés à l'administration d'oxybutynine racémique, qui comporte une quantité thérapeutiquement efficace de R(-)-4-diéthylamino-2-butynyle cyclohexylphénylglycolate ou d'un sel de qualité pharmaceutique ou de solvate de ce dernier, essentiellement dépourvu de son (S)-énantiomère correspondant.

2. Utilisation de R(-)-4-diéthylamino-2-butynyle cyclohexylphénylglycolate, y compris des sels de qualité pharmaceutique ou des solvates de ces derniers, dans la fabrication d'une composition pharmaceutique pour traiter les syndromes de l'intestin irritable (SII), et autres troubles de motilité impliquant le système gastro-intestinal, tout en réduisant le risque concomitant d'effets inhibiteurs cardiovasculaires indésirables associés à l'administration d'oxybutynine racémique, qui comporte une quantité thérapeutiquement efficace de R(-)-4-diéthylamino-2-butynyle cyclohexylphénylglycolate ou d'un sel de qualité pharmaceutique ou de solvate de ce dernier, essentiellement dépourvu de son (S)-énantiomère correspondant.

3. Utilisation de R(-)-4-éthylamino-2-butynyle cyclohexylphénylglycolate, y compris des sels de qualité pharmaceutique ou des solvates de ces derniers, dans la fabrication d'une composition pharmaceutique pour traiter l'incontinence urinaire, et autres troubles de motilité impliquant le système urétrogénital, tout en réduisant le risque concomitant d'effets inhibiteurs cardiovasculaires indésirables associés à l'administration d'oxybutynine racémique, qui comporte une quantité thérapeutiquement efficace de R(-)-4-éthylamino-2-butynyle cyclohexylphénylglycolate ou d'un sel de qualité pharmaceutique ou de solvate de ce dernier, essentiellement dépourvu de son (S)-énantiomère correspondant.

4. Utilisation de R(-)-4-éthylamino-2-butynyle cyclohexylphénylglycolate, y compris des sels de qualité pharmaceutique ou des solvates de ces derniers, dans la fabrication d'une composition pharmaceutique pour traiter le syndrome de l'intestin irritable (SII), et autres troubles de motilité impliquant le système gastro-intestinal, tout en réduisant le risque concomitant d'effets inhibiteurs cardiovasculaires indésirables associés à l'administration d'oxybutynine racémique, qui comporte une quantité thérapeutiquement efficace de R(-)-4-éthylamino-2-butynyle cyclohexylphénylglycolate ou d'un sel de qualité pharmaceutique ou de solvate de ce dernier, essentiellement dépourvu de son (S)-énantiomère correspondant.

5. L'utilisation de la revendication 1 ou 2 où le R(-)-4-diéthylamino-2-butynyle cyclohexylphénylglycolate, sel de qualité pharmaceutique, ou solvate de ce dernier, est configuré pour une administration par une voie sélectionnée dans le groupe de voies d'administration composé de : inhalation, parentérale, transdermique, rectale, sublinguale, ou orale.

6. L'utilisation de la revendication 3 ou 4 où le R(-)-4-éthylamino-2-butynyle cyclohexylphénylglycolate, sel de qualité pharmaceutique, ou solvate de ce dernier, est configuré pour une administration par une voie sélectionnée dans le groupe de voies d'administration composé de : inhalation, parentérale, transdermique, rectale, sublinguale, ou orale.

7. L'utilisation de la revendication 1 ou 2 où le R(-)-4-diéthylamino-2-butynyle cyclohexylphénylglycolate, sel de qualité pharmaceutique, ou solvate de ce dernier, est configuré pour une administration orale.

8. L'utilisation de la revendication 3 ou 4 où le R(-)-4-éthylamino-2-butynyle cyclohexylphénylglycolate, sel de qualité pharmaceutique, ou solvate de ce dernier, est configuré pour une administration orale.

9. L'utilisation de la revendication 1 ou 2 où le R(-)-4-diéthylamino-2-butynyle cyclohexylphénylglycolate, sel de qualité pharmaceutique, ou solvate de ce dernier, est configuré pour une administration orale à libération prolongée.

10. L'utilisation de la revendication 3 ou 4 où le R(-)-4-éthylamino-2-butynyle cyclohexylphénylglycolate, sel de qualité pharmaceutique, ou solvate de ce dernier, est configuré pour une administration orale à libération prolongée.

11. L'utilisation de la revendication 1 ou 2 où le R(-)-4-diéthylamino-2-butynyle cyclohexylphénylglycolate, sel de qualité pharmaceutique, ou solvate de ce dernier, est configuré pour une administration transdermique.

12. L'utilisation de la revendication 3 ou 4 où le R(-)-4-éthylamino-2-butynyle cyclohexylphénylglycolate, sel de qualité pharmaceutique, ou solvate de ce dernier, est configuré pour une administration transdermique.

13. L'utilisation de la revendication 1 ou 2 où la quantité de R(-)-4-diéthylamino-2-butynyle cyclohexylphénylglycolate, de sel de qualité pharmaceutique, ou de solvate de ce dernier, est suffisante pour fournir un dosage d'environ 1 mg à environ 200 mg par jour.

14. L'utilisation de la revendication 3 ou 4 où la quantité de R(-)-4-éthylamino-2-butynyle cyclohexylphénylglycolate, de sel de qualité pharmaceutique, ou de solvate de ce dernier, est suffisante pour fournir un dosage d'environ 1 mg à environ 200 mg par jour.
